# EUROPEAN PATENT APPLICATION

(11) **EP 2 386 552 A1**
(43) Date of publication of application: **16.11.2011**
(21) Application number: 11003836.1
(22) Date of filing: 10.05.2011
(51) Int. Cl.: C07D 339/04

(54) **Process for the preparation of (R) a-lipoic acid salts, their formulation and use in pharmaceutical compositions, in form of tablets containing them**

(30) Priority: 11.05.2010 IT MI2010 831
(71) Applicant: CBB Net S.A., 6900 Lugano (CH)
(72) Inventor: Borsa, Massimiliano, 20190 Virmodrone (MI) (IT); Cantabene, Carlo, 6900 Lugano (CH)
(74) Representative: Beneduce, Gianna

(57) **Abstract**

A process for the preparation of salts of (R) a-lipoic acid of formula (I), wherein the nitrogen organic base is selected among: piperazine, cysteamine and its corresponding disuiphide, cystamine, and diazabicyclo 2,2,2] octane and the corresponding pharmaceutical composition in form of tablets, that contains it as active ingredient, is described.

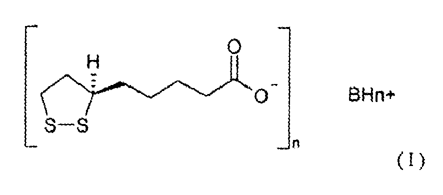

## Description

### State of the art

The α-lipoic acid, or thioctic acid, is known and widely utilized in therapy for its numerous properties. The presence, in its structure, of an asymmetric center on the carbon atom in position 3 of the dithiolane gives rise to two corresponding (R) and (S) enantiomers, which have shown to possess different interesting pharmacological properties.

Are known in the art numerous publications referred to α-lipoic acid: injectable solutions containing, as active ingredient, α-lipoic acid in form of salt with trometamol or with basic aminoacids are described and claimed in European Patent n° 0318891.

US Patent n° 6,271,254 describes pharmaceutical compositions which contain α-lipoic acid, in its (R) or (S) enantiomeric forms, as such or in form of pharmaceutically acceptable salts; said compositions, containing (R) and (S) α-lipoic acid salts proved to possess anti-inflammatory and analgesic activity in a higher degree when compared to the activity shown by the α-lipoic acid as well as by its enanthiomeric forms. As substances capable to supply, with (R) or (S) α-lipoic acid, pharmacologically acceptable salts, several compounds are cited, such as ammonium hydroxide, basic aminoacids, optionally substituted with alkyl- or oxyalkylamines, alkylendiamines, saturated cyclic amino compounds, N-methylglucamine, creatine and tromethamine. Among the saturated cyclic amino compounds, piperidine, piperazine, pirrolidine and morpholine are specifically cited, but the preparation of only an injectable solution of tromethamine (R) or (S) α-lipoate of these salts is cited.

US Patent Appln. 2004/0225007 A1 describes the preparations of two new forms of the trometamol salt of (R) thioctic acid which have different physicochemical properties.

WO 2010/151008 A2 describes a preparation of piperazine dithioctate by reacting together solutions in acetone of thioctic acid and of piperazine. A generic description of pharmaceutical compositions useful in the treatment of diabetic multiple neuritis and other pathologies, containing as active ingredient, piperazine dithioctate suitably formulated in capsule, granules, powder, emulsions, suspensions, syrups etc. is also generically indicated therein.

Pharmaceutical compositions in form of tablets are only referred to the α-lipoic acid, in its (R) or (S) enantiomeric form.

In fact, α-lipoic acid, and even more its (R) enantiomer, is unstable to the light, temperature and humidity and undergoes a physicochemical transformation during the compression, that reduces the release from the tablets in gastric ambient and probably this is one of the reasons of its poor oral absorption. Tablets having the same quali-quantitative composition of common excipients, like lactose monohydrate, talk, magnesium stearate and the like, have a dissolution velocity and a total release in acid ambient that depends on the drift compression force, verified by the breaking load of the tablet, and is different for the (R) α-lipoic acid and for its salts.

### Description of the invention

It has been now found, and this is the object of the present invention, that the (R) α-lipoic acid salts having the structure formula: 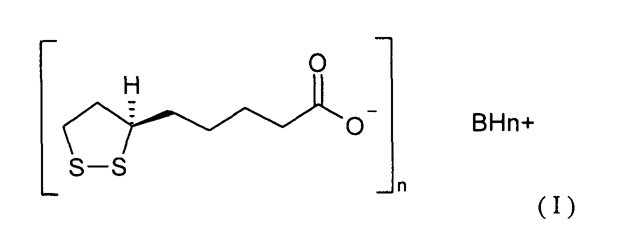 wherein:
B represents a nitrogen organic base pharmacologically acceptable selected in the group formed by piperazine, cysteamine and its corresponding disulphide cystamine, and
diazobicyclo [2,2,2] octane; n is an integer selected between 1 and 2, have interesting physicochemical properties the effects of which positively affect the solution profile of the tablets containing them.

As to the piperazine salt, piperazine (R) α-lipoate, the only name of which is cited in the above mentioned US Patent n° 6,271,254, can be prepared as mono salt (1:1) having a theoretical (R) α-lipoic acid content equal to 70.5%, or as double salt (2:1), having a theoretical (R) α-lipoic acid content equal to 82.7%. The piperazine monolipoate (1:1) has a high solubility in volatile solvents with low dielectric constant, such as ethanol and acetone, therefore it is practically impossible to crystallize it: its preparation requires the solvent evaporation or, in case of water, the lyophilizing, with formation of an amorphous product not suitable for the preparation of tablets by direct compression or powder mixtures. The double piperazine lipoate (2:1) crystallizes from volatile solvents with low dielectric constant, such as ethanol, acetone; from the ethanol, said salt precipitates in high density crystalline form, while from the acetone it is obtained in a very low density amorphous form, not at all suitable for the preparation of tablets by direct compression. The (R) α-lipoic acid salts with cysteamine and diazobicyclo [2,2,2] octane are crystalline with a (R) α-lipoic acid content of about 60% and 80% respectively.

The tablets containing the (R) α-lipoic acid salts are characterized by a content of the active principle higher than 60%, and have a dissolution profile quicker and more complete referred to the one of the (R) α-lipoic acid as such. The (R) α-lipoic acid, in the same composition and compression conditions, forms a gummy mass, due to a crystal modification and/or polymerization, that slows down the dissolution velocity and reduces the total release in an acid ambient simulating the gastric ambient. This effect is prevailing on any other effect and does not help to modify the excipients, for example, to change the filler compressibility or to add a crumbly agent.

The type of the employed raw material, acid or salt, requires the control of the compression pressure in order not to limit the dissolution velocity of the active principle: the dissolution pressure/velocity graphs of different salts in equal composition, form and weight tablets, show the non equilvalence of the (R) α-lipoic acid and its different salts. The tablets containing (R) α-lipoic acid as piperazine salt (2:1), the (R) α-lipoic acid cystamine salt and the (R) α-lipoic acid diazobicyclo [2,2,2] octane salt show a dissolution profile better than the one of the tablets containing other known salts.

### Detailed description of the invention

The (R) α-lipoic acid salts object of the present invention are prepared by mixing stoichiometric quantities of (R) α-lipoic acid and of the selected base in a suitable solvent and heating the mixture at a temperature from 25 to 60°C, preferably from 40 to 60°C, with a maintenance period of highest temperature normally lower than one hour, till obtaining a homogeneous solution which represents a guarantee of a complete salification. Should the base to be used be not available as such, but in the form of a corresponding salt, the base is first displaced from it according to traditional methods.

The obtained solution is then cooled down to a temperature between the ambient temperature and -15°C, preferably between 0 and -5°C, till crystallization of the desired salt, which is separated by filtration or centrifugation, washed with the same solvent and dried in vacuo at the maximum temperature of 55°C.

As solvents, 1-4 carbon atom aliphatic alcohols are used, having linear or branched chain, pure or denaturated by usual denaturants, optionally in a mixture with water or among them or with the mother liquors coming from a previous production cycle that used the same raw materials. The use of ketones, like acetone, as such, (Ex.7, 9) or in a mixture with some of the above mentioned alcohols (Ex.11, 12), produces an amorphous precipitate not suitable to be utilized for the direct compression because of the powder very low density. For industrial purpose a powder having low density (<0,2 g/mL) requires an additional operation (compactation / dry granulation / humid granulation) to produce tablets.

In alternative, the salification can be carried out dissolving separately, the (R) α-lipoic acid in a sufficient quantity of the solvent, or of a mixture of the above mentioned solvents, and the selected organic base in another solvent quantity or in a solvent mixture quantity, equal or different from the previous one, joining then together the two obtained solutions. The following Examples have the purpose of disclosing the invention in a higher detail without, in no way, limiting it.

### Example 1

### Trometamol (R) α-lipoate

Grams 82.4 (0.4 moles) of (R) α-lipoic acid and 48.4 g (0.4 moles) of trometamol are suspended in 300 mL absolute ethanol. The mixture is put under stirring and heated to 55-60°C till obtaining a homogeneous, clear solution. The solution is filtered, slowly cooled down to +5 - 0°C and maintained at this temperature for 4 or 5 hours, then the crystallized solid is collected on buckner and washed with little cold absolute ethanol. After drying in vacuo at 40°C, 121.0 g (0.372 moles equal to 93.1% of the theoretical value) of trometamol (R) α-lipoate are obtained.

### Example 2

### Trometamol (R) α-lipoate

Operation is carried out as in Example 1, using 0.2 moles of (R) thioctic acid and replacing absolute ethanol with 170 mL 96% ethanol. Grams 58.44 (0.18 moles equal to 89.3% of the theoretical value) of trometamol (R) α-lipoate are obtained.

### Example 3

### Trometamol (R) α-lipoate

Operation is carried out as in Example 2, using, as solvent, the mother liquor coming from the production cycle therein disclosed. Grams 62.9 (0.19 moles equal to 96% of the theoretical value) of trometamol (R) α-lipoate are obtained.

### Example 4

### Trometamol (R) α-lipoate

Operation is carried out as in Example 2, using 170 mL of 96% ethanol denaturated with 2% of acetone. Grams 57.0 of trometamol (R) α-lipoate (0.17 moles equal to 87.15% of the theoretical value) are obtained.

### Example 5

### Trometamol (R) α-lipoate

Operation is carried out as in Example 2, using as solvent, the mother liquors coming from the production cycle disclosed in Example 4. Grams 63.43 (0.194 moles equal to 96.99% of the theoretical value) are obtained.

### Example 6

### Cystamine (R) α-lipoate

Grams 56.3 of cystamine dihydrochloride (0.25 moles) and 50.6 g of triethyilamine (0.5 moles) are added to 350 mL of 96% ethanol and it is heated, under stirring and reflux, till obtaining a clear solution. It is cooled to below 60°C and 103.1 g of (R) thioctic acid (0.5 moles) are added, then stirred at 55 - 60°C till obtaining a clear solution (about 15min) and cooled between 0 and -5°C maintaining this temperature overnight. The crystallized solid is collected on buckner, washed with little 96% ice-cold ethanol. It is dried in vacuo at 40°C. Grams 120.5 g (0.21 moles equal to 85.3% of the theoretical value) of cystamine (R) α-lipoate are obtained.

### Example 7

### Diazobicyclo [2,2,2] octane (R) α-lipoate

Grams 10.31 of (R) thioctic acid (0.05 moles) and 2.80 g (0.025 moles) of diazobicyclo [2,2,2]octane are added to 35 mL of acetone and kept under stirring at 55°C till complete solution. It is concentrated in vacuo till a volume of 30 mL and cooled to between 0-5°C maintaining the temperature overnight.

The crystallized solid is collected on buckner and washed with little ice-cold acetone. It is dried in vacuo at 40°C obtaining 9.86 g of diazobicyclo [2,2,2]octane (R) α-lipoate (0.19 moles equal to 75.15% of the theoretical value).

### Esempio 8

### Piperazine (R) α-lipoato (2:1)

Grams 103.1 (0.5 moles) of (R) thioctic acid and 21.5 g (0.25 moles) of anhydrous piperazine are dissolved at 55 - 60°C in 300 mL of 96% ethanol. It is cooled to ambient temperature and then between 0 and -5°C and maintained overnight. The crystallized solid is collected on buckner, washing with little ice-cold ethanol. It is dried in vacuo at 40°C and 103.7 g of piperazine (R) α-lipoate (2:1) (0.21 moles equal to 84% of the theoretical value) are obtained.

### Example 9

### Piperazine (R) α-lipoate (2:1)

Operation is carried out as in Example 8, using, as solvent, 300 mL of acetone and dissolving first the anhydrous piperazine then the (R) thioctic acid. The mixture is stirred for a short time under reflux then cooled to ambient temperature, maintaining at said temperature for 4 hours. The precipitate is collected on buckner, washed with acetone, then dried in vacuo at 40°C obtaining 117.7 g of piperazine (R) α-lipoate (2:1) (0.236 moles equal to 94.5% of the theoretical value).

### Example 10

### Piperazine (R) α-lipoate (2:1)

Operation is carried out as in Example 9, using, as solvent, 300 mL of isopropanol. Grams 114.1 of piperazine (R) α-lipoate (2:1) (0,23 moles equal to 91.6% of the theoretical value) are obtained.

### Example 11

### Piperazine (R) α-lipoate (2: 1)

Grams 2.15 (0.025 moles) of anhydrous piperazine are dissolved, at ambient temperature, in 25 mL of 96% ethanol. Grams 10.31 (0.05 moles) of (R) thioctic acid are dissolved, at ambient temperature, in 25 mL of acetone. The acid solution is poured into the piperazine solution under a thorough stirring; the mixture is shortly refluxed and cooled to 0 /-5°C, maintaining at this temperature overnight. The crystallized solid is collected on buckner and washed with acetone. It is dried in vacuo at 40°C and 11.50 g (0.023 moles equal to 92.3% of the theoretical value) of piperazine (R) α-lipoate (2:1) are obtained.

### Example 12

### Piperazine (R) α-lipoate (1:1)

Grams 2.15 (0.025 moles) of anhydrous piperazine are dissolved, at ambient temperature, in 25 mL of 96% ethanol. Grams 5.16 (0.025 moles) of (R) thioctic acid are dissolved, at ambient temperature, in 10 mL of acetone. The acid solution is poured into the piperazine solution under a thorough stirring; the mixture is shortly refluxed and cooled to 0 / -5°C, maintaining at this temperature overnight. The salt does not precipitate and the solution is evaporated to dryness, in vacuo, at 40°C: the residue consists of a yellow spongy mass. Grams 6.04 (0.023 moles equal to 82.5% of the theoretical value) of piperazine (R) α-lipoate (1:1) are obtained.

### Example 13

### Piperazine (R) α-lipoate (1:1)

Grams 4.30 (0.05 moles) of anhydrous piperazine and 10.31 g (0.05 moles) of (R) thioctic acid are dissolved, at 55 - 60°C, in 30 mL of 96% ethanol. The mixture is cooled to ambient temperature, then to 0 / -5°C and kept overnight. The solution is evaporated to dryness, under vacuum, at 40°C. Grams 12.25 (0.042 moles equal to 83.7% of the theoretical value) of piperazine (R) α-lipoate (1:1), having the appearance of a yellow non-crystalline mass, are obtained.

**Table 1**

| | Ex. 8 | Ex. 12 | Ex. 6 | Ex. 1 | Ex. 7 |
|---|---|---|---|---|---|
| Appearance | yellow crystalline powder | yellow amorphous mass | yellow crystalline powder | yellow crystalline powder | yellow crystalline powder |
| m.p. | 105°C | ≈ 60°C | 176°C | 116°C | 69°C |
| pH 1 % in water | 5.5 | 7.2 | 6.6 | 6.6 | 5.2 |
| Specific rot. power | + 114.3° | + 80.3° | + 113.6° | + 117.2° | + 0.107° |
| HPLC % title | 82.25 | 58.6 | 67.9 | 63.2 | 80.2 |
| Expected HPLC % title | 82.7 | 70.5 | 73.9 | 63.1 | 78.62 |
| Powder apparent density | 0.5-0.6 | Not determinable | 0.5-07 | 0.5-0.7 | 0.4-0.6 |

Here below are given some examples of tablet formulations according to the invention.

### Example 14

| | |
|---|---|
| (R) α-lipoic acid | 600 mg |
| lactose | 450 mg |
| microcrystalline cellulose | 160 mg |
| sodium croscarmellose | 100 mg |
| talk | 20 mg |
| magnesium stearate | 60 mg |
| Tablet: capsule size 21 x 10mm. | |

### Example 15

| | |
|---|---|
| trometamol (R) α-lipoate (equal to 600mg of acid) (obtained as in Example 1) | 950 mg |
| lactose | 100 mg |
| microcrystalline cellulose | 160 mg |
| sodium croscarmellose | 100 mg |
| talk | 20 mg |
| magnesium stearate | 60 mg |
| tablet: capsule size 21 x 10mm. | |

### Example 16

| | |
|---|---|
| Piperazine (R) α-lipoate (equal to 600mg of acid) (obtained as in Example 8) | 726 mg |
| lactose | 324 mg |
| microcrystalline cellulose | 160 mg |
| sodium croscarmellose | 100 mg |
| talk | 20 mg |
| magnesium stearate | 60 mg |
| tablet: capsul size 21 x 10mm. | |

### Example 17

| | |
|---|---|
| cystamine (R) α-lipoate (equal to 580 mg of acid) (obtained as in Example 6) | 1000 mg |
| lactose | 100 mg |
| microcrystalline cellulose | 160 mg |
| sodium croscarmellose | 100 mg |
| talk | 20 mg |
| magnesium stearate | 60 mg |
| tablet: capsule size 21 x 10mm. | |

### Release profile

The release test was carried out according to Eur. Ph. 2.9.3 with a blade stirrer (100 rpm) in 1000 mL of artificial gastric buffer without pepsin (Eur. Ph. 1039900) using one tablet or a portion of it corresponding to no more than 300 mg of acid to guarantee solubility conditions. The title is carried out by drawing 10 mL of medium, filtering on paper then evaluating the acid content by the spectrophotometer at the wavelength of 330 nm versus standard.

The tablet hardness (N) is evaluated by a Schleuninger 6D durometer.

The compression force was settled to obtain three intervals of tablet hardness: 40-90 N; 90-120Ne> 130N.

**Table 2 : release of (R) α-lipoic acid tablet**

| Time (min) | Hardness 40-90 N | Hardness 90-120 N | Hardness 130-150 N |
|---|---|---|---|
| 0 | 0.00 | 0.00 | 0 |
| 10 | 26.45 | 24.96 | 8.94 |
| 20 | 54.01 | 48.78 | 10.02 |
| 30 | 74.62 | 62.24 | 9.92 |
| 50 | 90.27 | 71.05 | 11.34 |

### Diagram 1

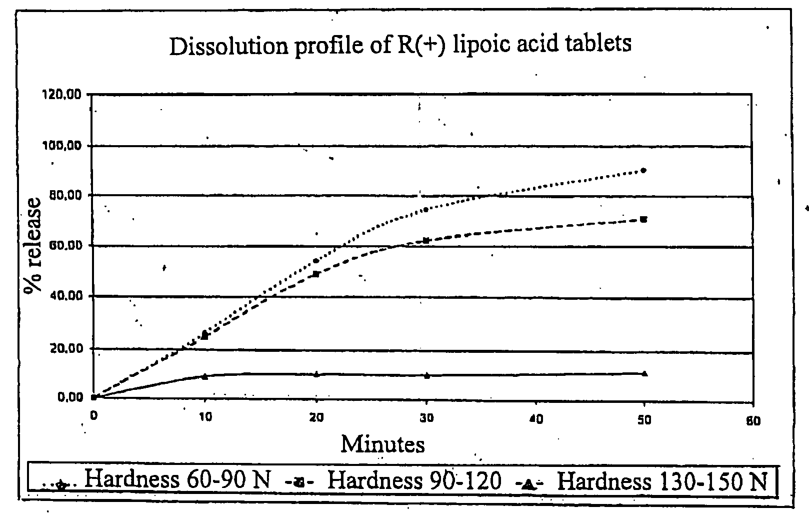

**Table 3 : release of trometanol (R) α-lipoate tablet**

| Time (min) | Hardness 40-90 N | Hardness 90-120 N | Hardness 130-150 N |
|---|---|---|---|
| 0 | 0.00 | 0.00 | 0.00 |
| 10 | 68.22 | 46.55 | 13.72 |
| 20 | 86.06 | 70.47 | 30.43 |
| 30 | 96.43 | 75.21 | 53.60 |
| 50 | 101.79 | 79.68 | 73.80 |

### Diagram 2

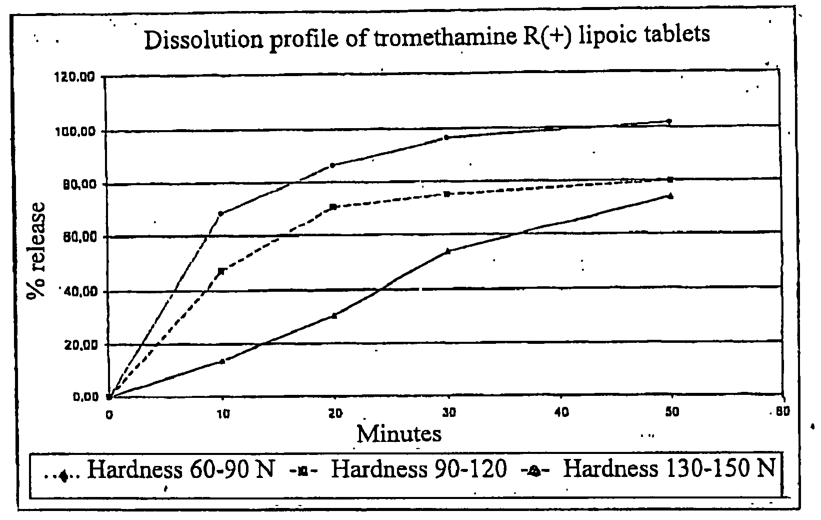

**Table 4 : release of piperazine (R) α-lipoate tablet**

| Time (min) | Hardness 40-90 N | Hardness 90-120 N | Hardness 130-150 N |
|---|---|---|---|
| 0 | 0.00 | 0.00 | 0.00 |
| 10 | 74.08 | 62.30 | 16.33 |
| 20 | 94.88 | 85.83 | 29.46 |
| 30 | 101.90 | 100.71 | 48.59 |
| 50 | 102.62 | 103.01 | 65.23 |

### Diagram 3

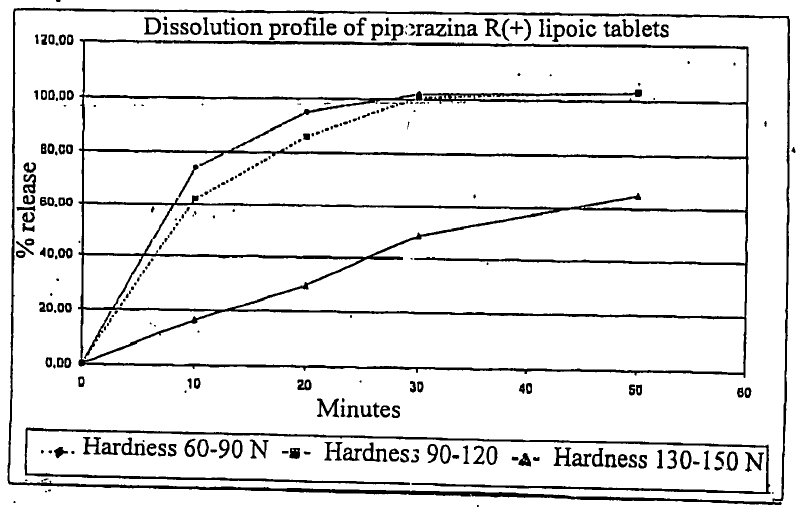

**Table 5 : release of cystamine (R) α-lipoate tablet**

| Time (min) | Hardness 40-90 N | Hardness 90-120 N |
|---|---|---|
| 0 | 0.00 | 0.00 |
| 10 | 63.01 | 38.60 |
| 20 | 86.06 | 62.70 |
| 30 | 96.43 | 78.60 |
| 50 | 101.79 | 85.40 |

### Diagram 4

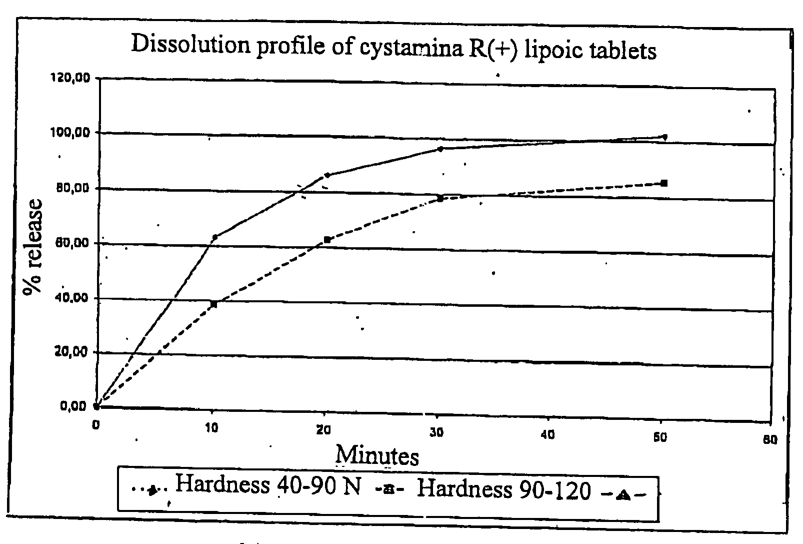

### Stability

The salts object of the present invention are much more stable as to regards the exposure to temperature and relative humidity (40°C - 75% R.H.) than the enantiomers and the racemate

### Diagram 5

Stability at 40°C

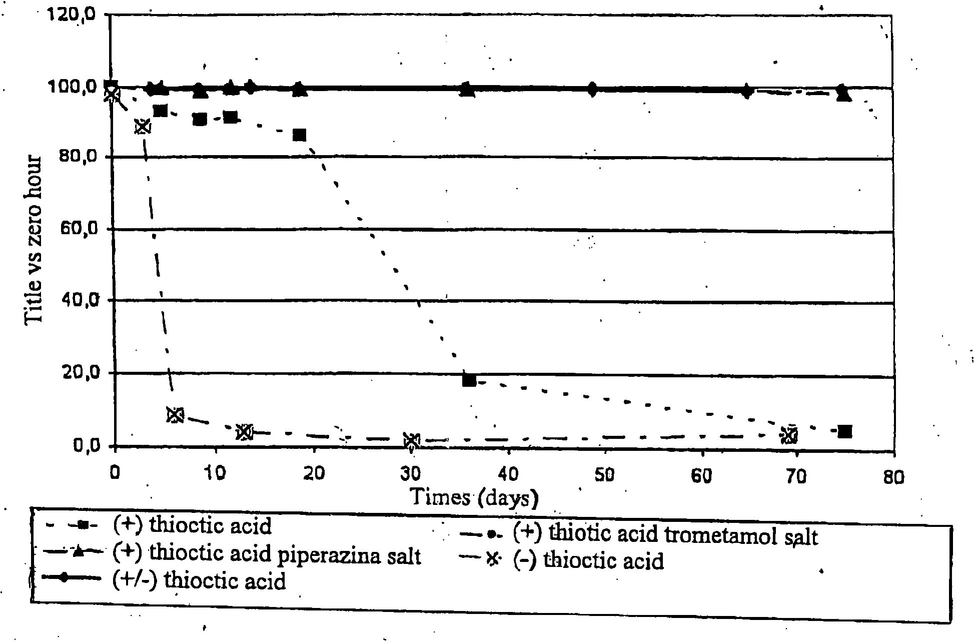

### Diagram 6

Stability at 40°C / 75% R.H.

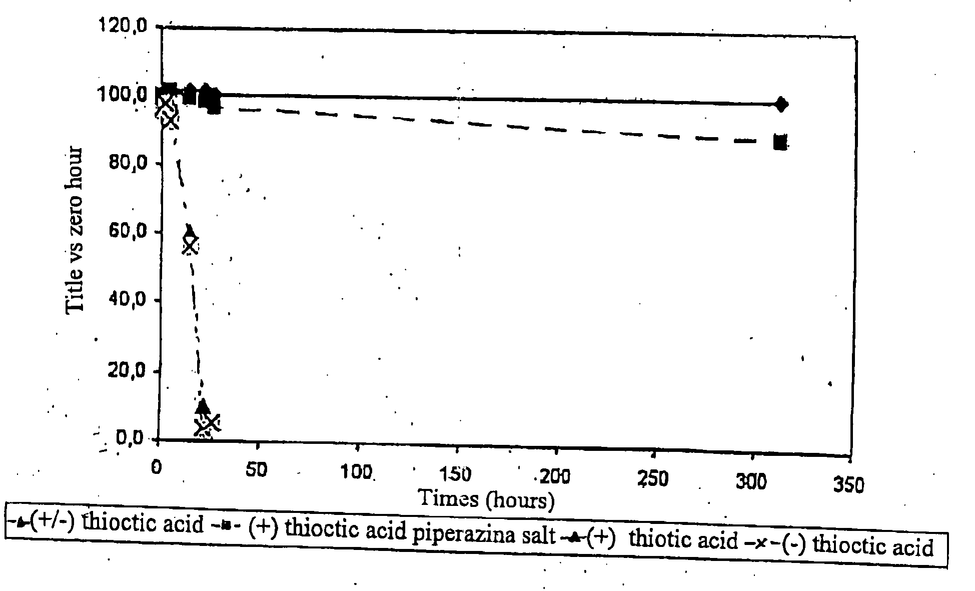

### Bioavailability

Tests carried out on (R) α-lipoic acid piperazine salt formulated in tablet demonstrated a better absorption and bioavailability than the corresponding tromethamine salt and lipoic acid as a racemate when administered at equivalent dosis.

## Claims

1. A process for the preparation of a (R) α-lipoic acid salt having the structure formula: 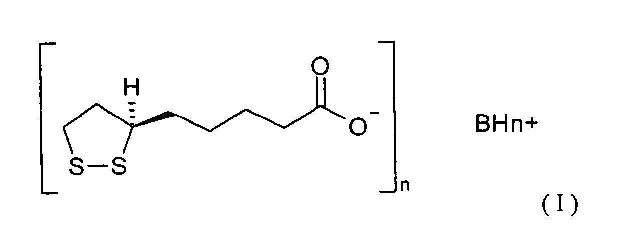 wherein:
B represents a pharmacologically acceptable nitrogen organic base selected in the group formed by piperazine, cysteamine and its corresponding disulphide, cystamine and diazabicyclo [2,2,2] octane;
n is an integer selected between 1 and 2 and
the corresponding pharmaceutical composition, in form of tablet, that contains said salt as active ingredient, **characterized by** the fact that stoichiometric quantities of (R) α-lipoic acid and of the above mentioned nitrogen base are mixed in an aliphatic alcohol of from 1 to 4 carbon atoms, having a linear or branched chain or in an aqueous mixture of said aliphatic alcohols and that the mixture is heated to a temperature of from 25 to 60°C for a time shorter than one hour to obtain a homogenous solution which is then cooled to a temperature of from 20 to -15°C to provide a crystalline precipitate having high density which is separated, washed with the same solvent and dried in vacuo at a temperature not higher than 55°C to provide the desired salt of formula (1) and that said salt is further formulated with suitable traditional excipients in a pharmaceutical composition in form of tablet by means of direct compression.

2. The process according to claim 1, **characterized by** the fact that the crystalline compound which separates has a density of about 0.5 - 0.6 g/mL and is suitable for the preparation of tablets by direct compression..

3. The process according to claims 1 and 2, **characterized by** the fact that the temperature to which the mixture is heated is comprised between 40 and 60°C and that the temperature to which the solution is cooled is comprised between 0 and -5°C.

4. The process according to claims 1-3, **characterized by** the fact that the nitrogen organic base is selected in the group consisting of piperazine, cisteamina, cistamina and diazobicyclo[2,2,2] octane and n is represented by 2.

5. A pharmaceutical composition for the oral administration in form of tablet, **characterized by** the fact that it contains a (R) α-lipoic acid salt obtained according to the previous claims.

6. The pharmaceutical composition according to claim 4, **characterized by** the fact that the salt of the (R) α-lipoic acid is the (R) α-lipoic acid piperazine salt (2:1).
